# EUROPÄISCHE PATENTSCHRIFT

(11) **EP 4 097 476 B1**
(45) Veröffentlichungstag und Bekanntmachung des Hinweises auf die Patenterteilung: **14.08.2024**
(21) Anmeldenummer: 21703156.6
(22) Anmeldetag: 25.01.2021
(51) Int. Cl.: G01N 33/531, G01N 33/543, G01N 33/545

(54) **DIAGNOSTISCHES REAGENZ ZUR QUANTITATIVEN BESTIMMUNG VON PROCALCITONIN IN EINER PROBE**
DIAGNOSTIC REAGENT FOR QUANTITATIVE DETERMINATION OF PROCALCITONIN IN A SAMPLE
RÉACTIF DE DIAGNOSTIC POUR LA DÉTERMINATION QUANTITATIVE DE LA PROCALCITONINE DANS UN ÉCHANTILLON

(30) Priorität: 28.01.2020 DE 102020102038
(43) Veröffentlichungstag der Anmeldung: 07.12.2022
(73) Patentinhaber: DiaSys Diagnostic Systems GmbH, 65558 Holzheim (DE)
(72) Erfinder: MASETTO, Thomas, 65582 Diez (DE); GRIMMLER, Matthias, 65604 Elz (DE)
(74) Vertreter: WSL Patentanwälte Partnerschaft mbB
(86) Internationale Anmeldenummer: PCT/EP2021/051577
(87) Internationale Veröffentlichungsnummer: WO 2021/151820

(56) Entgegenhaltungen:
- EP-A2- 1 111 050
- CN-A- 103 558 400
- CN-A- 105 891 497
- CN-A- 109 725 160
- CN-A- 109 959 797
- ANONYMOUS: "Covalent Coupling", 19 March 2013 (2013-03-19), pages 1 - 9, XP093076590, Retrieved from the Internet <URL:https://www.bangslabs.com/sites/default/files/imce/docs/TechNote%20205%20Web.pdf> [retrieved on 20230828]
- JOÃO PINTO DA COSTA ET AL: "Bionanoconjugation for Proteomics applications - An overview", BIOTECHNOLOGY ADVANCES., vol. 32, no. 5, 1 September 2014 (2014-09-01), GB, pages 952 - 970, XP055437897, ISSN: 0734-9750, DOI: 10.1016/j.biotechadv.2014.04.013

## Beschreibung

Die vorliegende Erfindung betrifft ein diagnostisches Reagenz zur quantitativen Bestimmung von Procalcitonin in einer Probe, wobei das Reagenz eine wässrige Suspension von Polymerpartikeln mit kovalent daran gebundenen Antikörpern gegen Procalcitonin ist. Des Weiteren betrifft die vorliegende Erfindung ein Verfahren zur Herstellung eines solchen diagnostischen Reagenz.

Procalcitonin (PCT) ist eine Vorstufe des Hormons Calcitonin und gleichzeitig einer der wichtigsten Marker in der Sepsis-Diagnostik. PCT wird insbesondere in der post-operativen Sepsis-Kontrolle standardmäßig eingesetzt. Hintergrund ist, dass bei v.a. bakteriellen Infektionen nach Operationen Leber- und Fettzellen vermehrt PCT produzieren. In der täglichen Klinikroutine wird PCT aber nicht nur postoperativ sondern auch allgemein begleitend für die initiale Diagnose und Differenzierung einer durch Viren, Bakterien, Pilze und Protozoen/Parasiten verursachten Sepsis verwendet und anschließend bei der Verlaufskontrolle eingesetzt, d.h. bei der Wirksamkeitsüberprüfung der eingesetzten Medikation.

Sepsis hat mehrere Bereiche die diagnostisch relevant sind. Unter 0,5 ng/ml ist eine Sepsis unwahrscheinlich. Über 0,5 - 2 ng/ml ist eine Inflammation/Entzündung wahrscheinlich. Das Vorliegen einer Sepsis muss über erneute zeitversetzte Messung von PCT und ggf. weiterer Parameter abgeklärt werden. Über 2 - 10 ng/ml liegt eine Sepsis mit wahrscheinlich bakteriellem Hintergrund vor. Über 10 ng/ml liegt ein schwerer septischer Schock vor. Diese Unterteilung dient zur Charakterisierung von Sepsis und der daraus abzuleitenden Maßnahmen. Das ist speziell im Hinblick auf den Zeitfaktor essentiell.

Jede Stunde in der eine Sepsis nicht diagnostiziert wird bzw. eine um eine Stunde später erfolgende Diagnose erhöht sich die Mortalität statistisch um 7%. Daher ist Sepsis in allen Regionen der Welt die dritthäufigste Todesursache. Vor diesem Hintergrund sind an die Sepsis-Diagnostik extrem hohe Anforderungen im Hinblick auf Sensitivität und Reproduzierbarkeit zu stellen. Auch nach längeren Lagerzeiten müssen die bei der Diagnostik eingesetzten Reagenzien zuverlässige Ergebnisse liefern.

Aus dem Stand der Technik sind verschiedene Diagnostikverfahren auf der Basis von Immunassays bekannt, mit denen PCT im Blut eines Patienten quantitativ bestimmt werden kann. Dabei werden Antikörper gegen PCT auf Polymerpartikeln gebunden, und nach Umsetzung mit dem in der zu untersuchenden Probe enthaltenen PCT kann dann die Auswertung erfolgen.

CN 109 959 797 A, CN 109 725 160 A, CN 103 558 400 A und EP 1 111 050 A2 offenbaren Procalcitonin-Test-Kits mit einem Reagenz, welches Polymerpartikel mit Antikörpern gegen Procalcitonin enthält.

CN 105 891 497 beschreibt ein Procalcitonin-Test-Kit mit einem Reagenz, welches Goldpartikel mit Antikörpern gegen Procalcitonin enthält.

Beim CLIA-Verfahren (Chemilumineszenz-Immunassay) muss ein Chemilumineszenz-Reagenz-Komponente verwendet werden, damit die Auswertung über die auftretende Chemilumineszenz erfolgen kann. Hierfür ist ein spezielles Gerät erforderlich, bei dem in der Regel eine kontinuierliche Resuspendierung der Partikel erfolgen muss.

Beim PETIA-Verfahren (Particle Enahnced Turbidimetric Immuno Assay) erfolgt eine turbidimetrische Auswertung über die Abnahme der Transmission von Licht durch die Reaktionsflüssigkeit. Je mehr PCT in einer Probe enthalten ist, desto stärker sind die zwischen den mit Antikörpern besetzten Partikeln auftretenden Quervernetzungen, was zu einer stärkeren Trübung der Flüssigkeit und damit zu einer Abnahme der Transmission führt. Es ist also kein zusätzliches Chemilumineszenz-Reagenz erforderlich, und die Messung kann mit einem einfachen Photometer-System, wie es in praktisch jedem diagnostischen Labor vorhanden ist, erfolgen.

Für die Genauigkeit und Aussagekraft der turbidimetrischen Auswertung ist es ganz entscheidend, dass der Grad der sich durch die Umsetzung mit PCT einstellenden Trübung stets zuverlässig mit der quantitativen Menge an PCT in der Probe korreliert. Dies ist beispielsweise dann nicht gegeben, wenn die mit Antikörpern beladenen Partikel nach der Herstellung im Laufe der Zeit spontan/unspezifisch agglutinieren, sedimentieren oder sich ihre spezifische gegen PCT gerichtete Antigen-Bindefähigkeit verändert.

Es besteht daher ein Bedarf an einem diagnostischen Reagenz, das für die turbidimetrische Auswertung mit einem einfachen Photometer geeignet ist, und das eine hohe Sensitivität zur quantitativen Bestimmung von Procalcitonin in einer Probe aufweist, wobei das Reagenz eine wässrige Suspension von Polymerpartikeln mit kovalent daran gebundenen Antikörpern gegen Procalcitonin ist, bei dem auch nach längeren Standzeiten keine oder nur extrem geringe Neigung zur Agglutination/Sedimentation feststellbar ist und die spezifische Reaktivität der Partikel weitmöglichst unverändert bleibt.

Diese Aufgabe wird erfindungsgemäß dadurch gelöst, dass
- die suspendierten Polymerpartikel eine durchschnittliche Partikelgröße im Bereich von > 300 nm bis 450 nm aufweisen,
- die Suspension einen Anteil an darin gelöstem Zucker oder Zuckeralkohol im Bereich von 25 bis 250 g/l aufweist und
- die Suspension einen pH-Wert im Bereich von 8,5 bis 10 aufweist.

Durch die erfindungsgemäß vorgeschlagene Merkmalskombination wird ein diagnostisches Reagenz für die quantitative Bestimmung von PCT erhalten, das in dem hier maßgeblichen Bereich extrem geringer PCT-Konzentrationen einer Probe eine sehr hohe Sensitivität aufweist. Außerdem bleibt diese hohe Sensitivität auch bei längerer Lagerung über bis zu 24 Monate erhalten. Diese hohe Lagerstabilität ist insbesondere vor dem Hintergrund beachtlich, dass die erfindungsgemäß eingesetzten Polymerpartikel eine relativ hohe Partikelgröße von > 300 nm und mehr aufweisen.

Die hohe Partikelgröße der Polymerpartikel ist insbesondere im Hinblick auf die verlangte hohe Sensitivität des diagnostischen Reagenz ausdrücklich gewünscht. Die relativ großen Partikel haben eine entsprechend große Oberfläche, sodass hierauf eine entsprechend große Anzahl an Antikörpern gegen PCT kovalent gebunden werden kann. Es bestehen somit mehr potentielle Kopplungsstellen für die PCT-Moleküle, was zu einer erhöhten Sensitivität des diagnostischen Reagenz führt.

Die hohe Partikelgröße ist außerdem vorteilhaft im Hinblick auf die Lichtstreuungseigenschaften. Es hat sich herausgestellt, dass die "Lichtausbeute" mit den erfindungsgemäßen Polymerpartikeln deutlich besser ist. Bei gleichem Vernetzungsgrad erfolgt mit den erfindungsgemäßen Partikeln quasi eine Nachverstärkung des Signals. Je größer die Partikel desto stärker das Signal. Prinzipien dahinter sind Effekte wie die Rayleigh-Streuung, Mie-Streuung, etc. an kolloidalen Nanopartikeln in Suspension.

Überraschenderweise konnte mit der vorliegenden Erfindung erreicht werden, dass trotz der hohen Partikelgröße eine bessere Lagerstabilität erzielt wird als zu erwarten gewesen wäre. Üblicherweise neigen größere Partikel stärker zur Sedimentation und sind somit nach längerer Lagerzeit schlechter zu resuspendieren. Durch die erfindungsgemäß vorgeschlagene Kombination aus in der Suspension gelöstem Zucker bzw. Zuckeralkohol und dem in der Suspension einzustellenden pH-Wert, kann jedoch auch mit den relativ großen Partikeln der Erfindung überraschenderweise eine äußerst lagerstabile Suspension bei gleichbleibender Reaktivität erhalten werden.

Das hier beanspruchte diagnostische Reagenz dient zur quantitativen Bestimmung von PCT in einer Probe. Unter einer "Probe" wird im Zusammenhang mit der vorliegenden Erfindung jedes für Zwecke der Analyse aufbereitete Material verstanden, das einen zu analysierenden Anteil an PCT enthält. In den meisten Fällen wird es sich bei der Probe um eine Probe von frischem Vollblut handeln, die für Zwecke der Durchführung der Analyse gegebenenfalls entsprechend aufbereitet wurde. Von der vorliegenden Erfindung umfasst sind jedoch auch andere flüssige Proben, die PCT enthalten, wie z.B. Standardlösungen und Kalibratoren.

Das diagnostische Reagenz der vorliegenden Erfindung ist für die quantitative PCT-Bestimmung geeignet. "Quantitative Bestimmung" bedeutet in diesem Zusammenhang, dass aus der Menge an PCT-Molekülen, die an den Polymerpartikeln gebunden werden, Rückschlüsse auf die Menge an PCT in der Probe gezogen werden können.

Die Polymerpartikel liegen bei dem erfindungsgemäßen Reagenz in einer wässrigen Suspension vor. Der Begriff "wässrige Suspension" bezeichnet in diesem Zusammenhang eine Aufschlämmung der mit Antikörpern gegen Procalcitonin beladenen Polymerpartikel in Wasser bzw. einer wässrigen Lösung, in der Zucker, Zuckeralkohol und/oder Puffersubstanzen gelöst sind. Der Begriff "Suspension" ist im Zusammenhang mit der vorliegenden Erfindung eng auszulegen und bedeutet, dass nahezu alle Partikel in der wässrigen Phase schweben und nicht sedimentiert sind. Eine Suspension im Sinn der vorliegenden Erfindung liegt daher dann vor, wenn wenigstens 95 %, wenigstens 96 %, wenigstens 97 %, wenigstens 98 % oder gar wenigstens 99 % der Polymerpartikel frei schwebend vorliegen.

Die durchschnittliche Partikelgröße der Polymerpartikel liegt erfindungsgemäß im Bereich von > 300 nm bis 450 nm. Bei bestimmten Ausführungsformen innerhalb des beanspruchten Bereiches ist die durchschnittliche Partikelgröße vorzugsweise <410 nm oder <360 nm. Die Partikelgröße der Polymerpartikel wird im Falle der vorliegenden Erfindung anhand dynamischer Lichtstreuung bei 25°C bestimmt, z.B. mit dem Zetasizer Pro von Malvern. Die durchschnittliche Partikelgröße bezieht sich auf das Zahlenmittel.

Das Polymermaterial, aus dem die Polymerpartikel bestehen, ist vorzugsweise ausgewählt unter Acrylpolymer, Dextran-Epichlorhydrin-Copolymer, Polymethylmethacrylat, Polystyrol, Silica (Kieselgel) und Kombinationen hiervon. Bei bestimmten Ausführungsformen bestehen die Polymerpartikel durchgehend aus einem Polymermaterial oder Gemischen hiervon. Bei anderen Ausführungsformen bestehen die Partikel aus mehreren Schichten unterschiedlicher Polymere.

Bei speziellen Ausführungsformen können die Partikel einen Kern aufweisen und eine oder mehrere auf diesen Kern aufgeschichtete Schichten. Der Kern und eine oder mehrere darauf aufgeschichtete Schichten können aus einem Polymermaterial, verschiedenen Polymermaterialien oder einem nicht-polymerischen Material bestehen, vorausgesetzt dass die Polymerpartikel überwiegend aus Polymermaterial bestehen.

Ein Polymerpartikel im Sinne der vorliegenden Erfindung liegt dann vor, wenn die Partikel zu wenigstens 80 Gew.-%, wenigstens 90 Gew.-%, wenigstens 95 Gew.-% oder zu 100 Gew.-% aus einem Polymermaterial oder aus einer Kombination verschiedener Polymermaterialien bestehen.

Die Dichte des Polymerpartikels liegt insgesamt vorzugsweise im Bereich von 0,9 bis 1,1 g/cm³ und besonders bevorzugt im Bereich von 1,0 ± 0,5 g/cm³.

An der Oberfläche weisen die Polymerpartikel vorzugsweise funktionelle Gruppen auf, über die eine kovalente Bindung der Antikörper an der Oberfläche der Polymerpartikel erfolgen kann. Vorzugsweise sind diese funktionellen Gruppen ausgewählt unter einer Carboxylgruppe (-COOH), primären Amingruppe (-RNH₂), aromatischen Amingruppe (-ArNH₂), Chlormethylgruppe (-CH₂Cl), aromatischen Chlormethylgruppe (-ArCH₂Cl), Amidgruppe (-CONH₂), Hydrazidgruppe (-CONHNH₂), Aldehydgruppe (-CHO), Hydroxylgruppe (-OH), Thiolgruppe (-SH), Epoxygruppe und Biotin-Avidin.

Der wässrige Anteil der Suspension des erfindungsgemäßen diagnostischen Reagenz enthält darin gelöst einen Zucker- oder Zuckeralkohol-Anteil mit einer Konzentration im Bereich von 25 bis 250 g/l. Bei bestimmten Ausführungsformen liegt die Konzentration an Zucker- und/oder Zuckeralkohol im Bereich von 50 bis 200 g/l. Bei manchen Ausführungsformen beträgt die Konzentration 50 bis 150 g/l, bei anderen Ausführungsformen 150 bis 250 g/l.

Der Zucker oder Zuckeralkohol, der im wässrigen Anteil der Suspension gelöst ist, ist bei bestimmten Ausführungsformen ausgewählt unter Saccharose, Mannitol, Sorbitol, Xylitol, Maltitol, Raffinose, Rhamnose und Kombinationen hiervon. Sofern eine Kombination von Zuckern/Zuckeralkoholen eingesetzt wird, beziehen sich die obigen Mengenangaben auf die Summe der Anteile der Zucker/Zuckeralkohole dieser Kombination.

Der pH-Wert der wässrigen Suspension, in welcher die mit Antikörpern beladenen Polymerpartikel suspendiert sind, liegt im Bereich von 8,5 bis 10. Bei manchen Ausführungsformen liegt der pH-Wert im Bereich von 9,0 bis 10,0. Bei bestimmten Ausführungsformen ist der pH-Wert der Suspension > 9. Bei manchen Ausführungsformen liegt der pH-Wert der Suspension im Bereich von 9,1 bis 10,0. Bei bestimmten Ausführungsformen liegt der pH-Wert der Suspension bei 9,0 ± 0,5. Bei besonderen Ausführungsformen liegt der pH-Wert der Suspension bei 9,5 ± 0,1.

Das erfindungsgemäß beanspruchte diagnostische Reagenz zeichnet sich durch eine sehr hohe Lagerstabilität aus. Dies drückt sich unter anderem darin aus, dass die Trübung der Suspension auch nach mehreren Monaten im Vergleich zum Ausgangswert stabil ist. Insbesondere zeichnet sich die Suspension der vorliegenden Erfindung dadurch aus, dass die Absorption der Suspension bei 660 nm innerhalb von 90, 120, 150 oder gar 180 Tagen ab dem Zeitpunkt der Herstellung der Suspension um weniger als 5 % vom Ausgangswert am Tag Null abweicht. Bei bestimmten Ausführungsformen beträgt die Abweichung sogar innerhalb von 12 Monaten oder gar innerhalb von 24 Monaten weniger als 5 %. Bei bestimmten Ausführungsformen beträgt die Abweichung sogar weniger als 3 % oder gar weniger als 2 %. Bei bestimmten Ausführungsformen der Erfindung ist die geringe Abweichung im Trübungsgrad innerhalb der vorgenannten Zeiträume auch bei anderen Wellenlängen im Bereich von 340 bis 800 nm messbar.

Bei einer bestimmten Ausführungsform der vorliegenden Erfindung handelt es sich bei den an den Polymerpartikeln kovalent gebundenen Antikörpern um monoklonale Antikörper gegen PCT. Bei anderen Ausführungsformen liegen polyklonale Antikörper gegen PCT vor. Bei noch weiteren Ausführungsformen der Erfindung sind die Polymerpartikel mit kovalent daran gebundenen rekombinanten Antikörpern gegen PCT oder kovalent daran gebundenen Antikörper-Fragmenten gegen PCT beladen.

Mit der vorliegenden Erfindung wird auch ein Verfahren zur Herstellung eines diagnostischen Reagenz der oben beschriebenen Art beansprucht. Bei diesem Verfahren werden zunächst die Polymerpartikel mit den Antikörpern gegen PCT in Kontakt gebracht, wobei dies unter Bedingungen geschieht, bei denen eine kovalente Bindung der Antikörperan die Oberfläche der Polymerpartikel erfolgt. Die Beladung der Polymerpartikel mit den Antikörpern erfolgt in einer wässrigen Suspension über einen bestimmten Zeitraum bei einer bestimmten Temperatur und einem bestimmten pH-Wert.

Das erfindungsgemäße Verfahren ist dadurch gekennzeichnet ist, dass die kovalente Bindung der Antikörper über die funktionellen Gruppen an der Oberfläche der Polymerpartikel bei einem pH-Wert von 3 bis 6 erfolgt. Bei bestimmten Ausführungsformen erfolgt die kovalente Bindung bei einem pH-Wert von 3 bis 5. Bei bestimmten Ausführungsformen liegt der pH-Wert bei der Reaktion, die zur kovalenten Bindung der Antikörper über die funktionellen Gruppen an der Oberfläche der Polymerpartikel führt, bei 4,0 ± 0,5.

Die Einstellung des pH-Werts bei der Reaktion, die zur kovalenten Bindung der Antikörper über die funktionellen Gruppen an der Oberfläche der Polymerpartikel führt, erfolgt vorzugsweise unter Verwendung von anorganische oder organischen Puffersubstanzen, wie z.B. Borat, Citrat, Phosphat, Malat, Maleat, Succinat, Essigsäure/Acetat. Zum Einstellen/Justieren des pH-Werts wird vorzugsweise Salzsäure /Natronlauge verwendet.

Bei bestimmten Ausführungsformen des erfindungsgemäßen Verfahrens erfolgt die kovalente Bindung der Antikörper über die funktionellen Gruppen an der Oberfläche der Polymerpartikel bei einer Temperatur im Bereich von 20 bis 29 °C. Bei anderen Ausführungsformen erfolgt diese Reaktion im Bereich von 30 bis 34 °C oder gar im Bereich von 35 bis 45 °C.

Bei bestimmten Ausführungsformen des erfindungsgemäßen Verfahrens erfolgt die kovalente Bindung der Antikörper in einem der vorgenannten definierten pH-Bereiche und bei einer Temperatur, die in einem der oben definierten Bereiche liegt, über einen Zeitraum von 20 bis 80 Stunden. Bei bestimmten Ausführungsformen erfolgt die Reaktion über einen Zeitraum von > 30, > 40, > 50 oder gar > 60 Stunden.

Nach Ablauf der oben angegebenen Zeiträume wird der pH-Wert der Suspension, der nun mit Antikörpern beladenen Polymerpartikel auf den Bereich von 8 bis 10 abgesenkt. Hierfür kann eines oder mehrere der oben angegebenen Alkalisierungsmittel bzw. Puffersubstanzen zugegeben werden.

Die Einstellung des pH-Werts in der Suspension erfolgt vorzugsweise unter Verwendung von Puffersubstanzen mit Amingruppen, wie z.B. EPPS, HEPPS, Tricin, Tris, Glycylglycin, Bicin, TAPS, Borsäure, Ethanolamin, CHES, Glycin und CAPS. Zum Einstellen/Justieren des pH-Werts wird vorzugsweise Salzsäure /Natronlauge verwendet.

Die nach dem erfindungsgemäßen Verfahren erhaltenen Polymerpartikel, die mit Antikörpern oder Antikörper-Fragmenten beladen sind, weisen eine ausgesprochen hohe Sensitivität und eine sehr große Lagerstabilität auf, wie es zuvor bereits beschrieben wurde.

Für Zwecke der ursprünglichen Offenbarung wird darauf hingewiesen, dass sämtliche Merkmale, wie sie sich aus der vorliegenden Beschreibung, den Figuren und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Des Weiteren wird darauf hingewiesen, dass es für den Fachmann selbstverständlich ist, dass die nachfolgenden Ausführungsbeispiele lediglich dazu dienen, mögliche Ausführungsformen der vorliegenden Erfindung beispielhaft anzugeben. Der Fachmann wird daher ohne Weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende explizite Darstellung sämtlicher denkbarer Ausführungsformen wird hier nur der Kürze und der Lesbarkeit der Beschreibung wegen verzichtet.

### Figuren

- Figur 1:: Ergebnisse der Untersuchung des Einflusses der Inkubationszeit auf die Sensitivität
- Figur 2:: Ergebnisse der Untersuchung des Einflusses des pH-Wertes auf die Sensitivität
- Figur 3:: Ergebnisse der Untersuchung der Lagerungsstabilität
- Figur 4:: Ergebnisse der Untersuchung der Lagerungsstabilität
- Figur 5:: Ergebnisse der Untersuchung des Einflusses der Partikelgröße auf die Messpräzision
- Figur 6:: Ergebnisse der Untersuchung des Einflusses der Partikelgröße auf die Empfindlichkeit und die Linearität
- Figur 7:: Ergebnisse der Untersuchung des Einflusses der Zuckerkonzentration auf die Kalibration
- Figur 8:: Ergebnisse der Untersuchung des Effekts unterschiedlicher Kombinationen von Saccharose und pH-Wert auf die Sensitivität
- Figur 9:: Ergebnisse der Untersuchung des Effekts unterschiedlicher Kombinationen von Saccharose und pH-Wert auf die Sensitivität
- Figur 10:: Ergebnisse der Untersuchung des Effekts unterschiedlicher Kombinationen von Saccharose und pH-Wert auf die Sensitivität
- Figur 11:: Ergebnisse der Untersuchung des Einflusses der Inkubationszeit auf die Sensitivität
- Figur 12: Ergebnisse der Untersuchung der Stabilität der Reaktivität bei einem pH von 8,1
- Figur 13: Ergebnisse der Untersuchung der Stabilität der Reaktivität bei einem pH von 9,0
- Figur 14: Ergebnisse der Untersuchung der Stabilität der Reaktivität bei einem pH von 9,5
- Figur 15: Ergebnisse der Untersuchung der Langzeit-Stabilität
- Figur 16: Ergebnisse der Untersuchung der Langzeit-Stabilität

### Ausführungsbeispiele

Im Folgenden werden verschiedene Vergleichsversuche präsentiert, bei denen unterschiedliche Parameter des Verfahrens zur Erzeugung des erfindungsgemäßen diagnostischen Reagenz variiert werden. Hierfür wurden Polymerpartikel aus Polystyrol unter verschiedenen Bedingungen mit Antikörpern gegen PCT beladen.

Bei einer Ausführungsvariante wiesen die Polymerpartikel als funktionelle Gruppe Carboxylgruppen (-COOH) auf und bei einer anderen Ausführungsvariante wiesen die Polymerpartikel als funktionelle Gruppe Chlormethylgruppen (-CH₂Cl) auf.

### I. Ausführungsvariante mit Chlormethylgruppen

### 1. Inkubationszeit

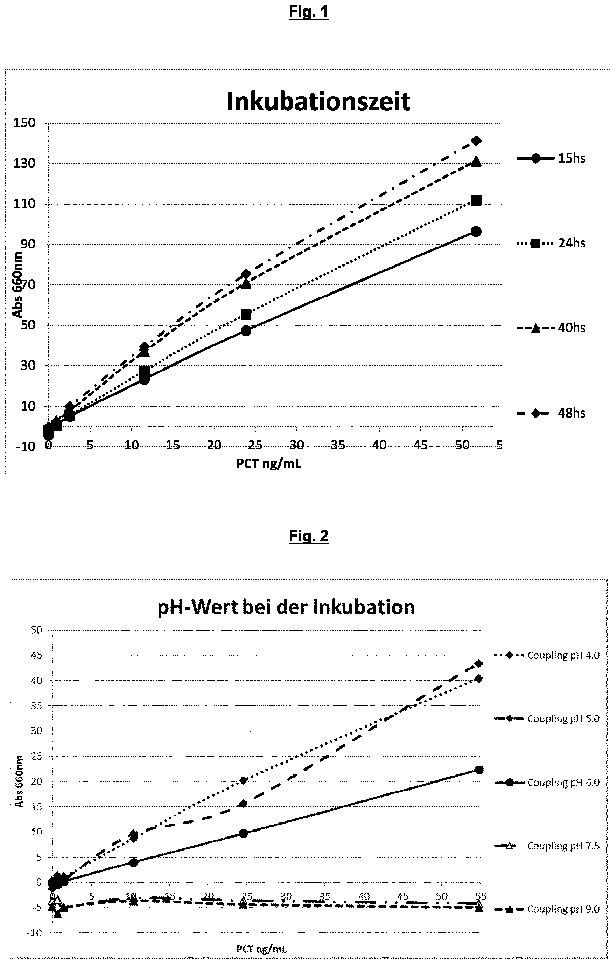

Die eingesetzten Polymerpartikel wiesen eine durchschnittliche Partikelgröße von > 350 nm auf. Die Partikel wurden über einen Zeitraum von 15 bis 48 Stunden mit den Antikörpern gekoppelt, und die Ergebnisse der verschiedenen Ansätze sind in Figur 1 dargestellt.

Ausgedrückt sind diese Ergebnisse anhand der Absorption bei 660 nm, und die vergleichsweise hohen Absorptionswerte des Ansatzes, bei dem die Inkubationszeit 48 Stunden betrug, belegen, dass die länger inkubierten Ansätze bei der jeweiligen Konzentration an PCT in der Probe eine signifikant höhere Sensitivität liefern, da im Vergleich zu den anderen Ansätzen offenbar höhere Anteile des in der Probe enthaltenen PCT gebunden werden, sodass auf diese Weise die Menge an in der Probe enthaltenem PCT quantitativ exakter bestimmt werden kann.

### 2. pH-Wert

Bei diesen Versuchen wurde der pH-Wert bei der Kopplungsreaktion im Bereich von 4 bis 9 variiert.

Die in Figur 2 dargestellten Ergebnisse belegen, dass bei niedrigeren pH-Werten eine bessere Beladungseffizienz erreicht wird. Es werden somit Polymerpartikel erreicht, die eine höhere Bindungskapazität gegenüber PCT und damit eine höhere Sensitivität aufweisen.

### 3. Lagerungsstabilität

Zur Überprüfung der Suspensionsstabilität des erfindungsgemäßen diagnostischen Reagenz wurde eine wässrige Suspension der Polymerpartikel mit einer Lehrprobe (physiologische Kochsalzlösung) versetzt und anschließend die Trübung des daraus erhaltenen Gemisches ermittelt. Die dabei eingesetzten Polymerpartikel waren unter verschiedenen pH-Bedingungen über einen Zeitraum von bis zu 120 Tagen gelagert worden.

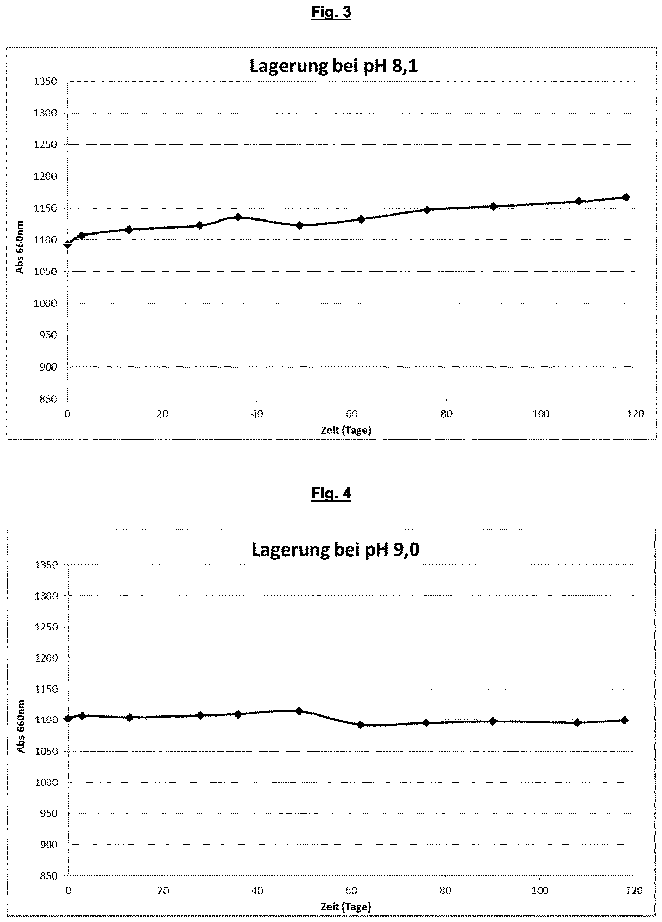

Die in den Figuren 3 und 4 dargestellten Ergebnisse belegen, dass bei einer Lagerung der Polymerpartikel bei einem pH-Wert von 9,0 insbesondere bei den Proben, die über 60 Tage und mehr gelagert worden waren, eine deutlich geringere Trübung auftritt als bei den Proben, die bei einem pH-Wert von 8,1 gelagert worden waren. Dies belegt eindeutig, dass die Neigung zur Agglutination und Sedimentation bei den Ansätzen deutlich geringer ist, die bei pH 9 gelagert werden.

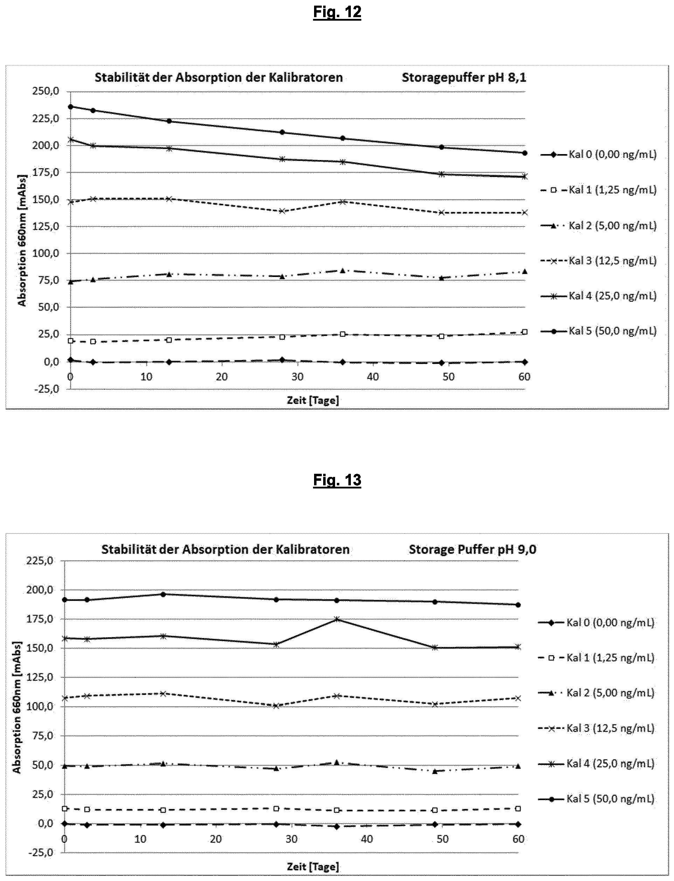

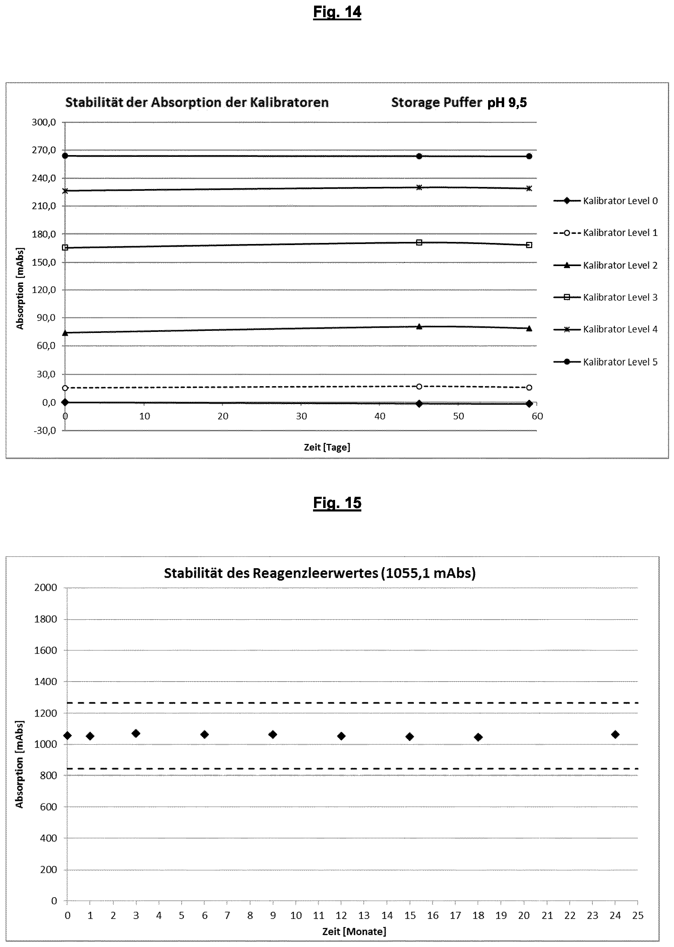

Wie den Figuren 12 bis 14 zu entnehmen ist, ist auch die Stabilität der Reaktivität im höheren pH-Bereich, nämlich im Bereich pH 9,0 (vgl. Figur 13) bis pH 9,5 (vgl. Figur 14) besser als im niedrigeren Bereich (vgl. Figur 12: pH 8,1). Im hier untersuchten Zeitraum von 60 Tagen, ist eine besonders hohe Stabilität der Reaktivität bei einem pH-Wert im Bereich > 9 feststellbar (vgl. Figur 14: pH 9,5).

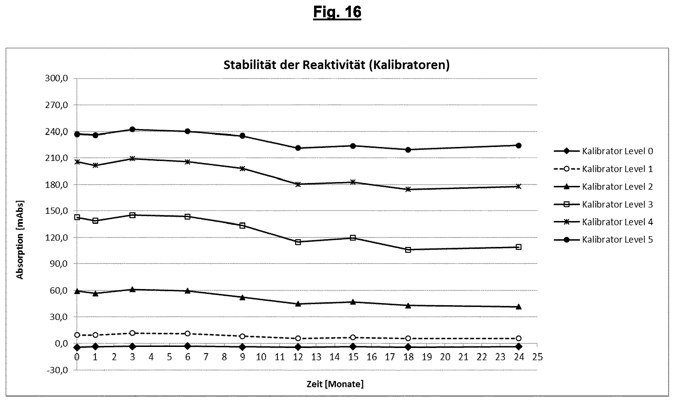

Die Figuren 15 und 16 belegen die hohe Langzeit-Stabilität von bis zu 24 Monaten bei einer Ausführungsform mit einem Lagerpuffer mit einem pH von 9,0. Bei den Ergebnissen zu der Untersuchung der Reaktivität der Kalibratoren erklärt sich der kleine Sprung in der Kurve bei 12 Monaten durch einen nach 12 Monaten erfolgten Wechsel der Lampe am Detektor.

### II. Ausführungsvarianten mit unterschiedlichen Partikelgrößen

Um den Einfluß der Partikelgröße zu untersuchen wurden Polymerpartikel mit Chlormethylgruppen, die eine durchschnittliche Partikelgröße von > 350 nm aufwiesen, mit Polymerpartikeln mit Chlormethylgruppen verglichen, die eine durchschnittliche Partikelgröße von < 300 nm aufwiesen.

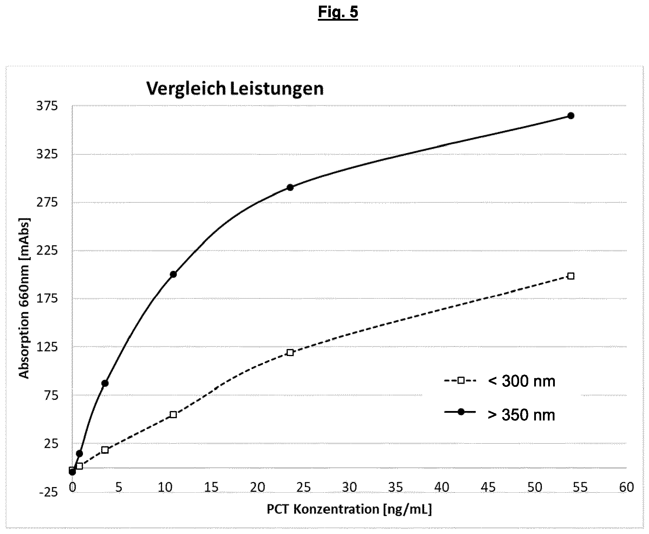

Figur 5 zeigt, dass die Reaktivität, besonders im Bereich von Konzentrationen 0,2-2 ng/mL, wo die zwei medizinisch relevanten Entscheidungsbereiche von Procalcitonin liegen, bei den Polymerpartikeln mit einer durchschnittlichen Partikelgröße von > 350 nm viel stärker ist als bei den Polymerpartikeln mit einer durchschnittlichen Partikelgröße von < 300 nm. Dadurch wird die Messpräzision signifikant verbessert.

Auch die Wiederfindung (Richtigkeit der Konzentration) ist mit den Polymerpartikeln mit einer durchschnittlichen Partikelgröße von > 350 nm besser als mit den Polymerpartikeln mit einer durchschnittlichen Partikelgröße von < 300 nm (siehe Tabelle 1 unten).

**Tabelle 1**

| | **Sollwert PCT** [ng/mL] | **Bereich** [ng/mL] | **Wiederfindung** < 300 nm | **Wiederfindung** > 350 nm |
|---|---|---|---|---|
| **Kontrolle Level 1** | 0,80 | 0,60 - 1,00 | 1,14-1,12 | 0,75-0,74 |
| **Kontrolle Level 2** | 10,85 | 8,68 - 13,03 | 10,58-10,57 | 10,46-10,24 |

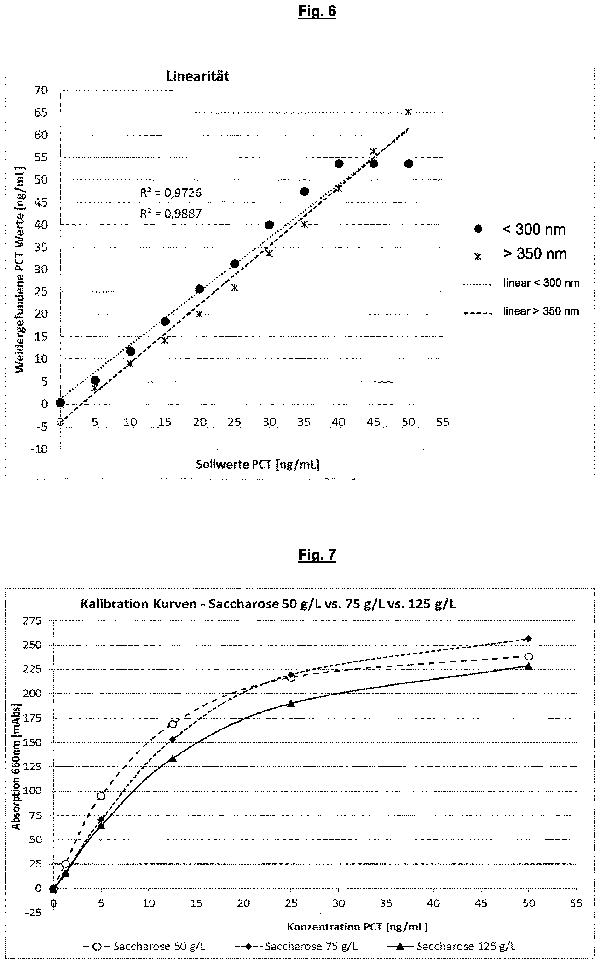

Figur 6 zeigt die Ergebnisse der Messung der Empfindlichkeit (als Präzision am Cut-Off gemessen) und die Linearität der Wiederfindung einer verdünnten Probe.

Mit den Polymerpartikeln mit einer durchschnittlichen Partikelgröße von < 300 nm kann nur bis zum Sollwert 35 ng/mL linear gemessen werden. Höhere Konzentrationen werden nicht mehr unterschieden.

Die Polymerpartikel mit einer durchschnittlichen Partikelgröße von > 350 nm haben bis 65 ng/mL eine lineare Wiederfindung. Der Korrelationskoeffizient der größeren Partikel ist dementsprechend auch höher als der der kleineren Partikel.

Auch die Wiederfindung und die Präzision von einer Probe am medizinisch relevanten Cut-off (0,5 ng/mL PCT) sind bei den größeren Partikeln deutlich besser als bei den kleineren (siehe Tabelle 2 unten).

**Tabelle 2**

| | **Gemessene Konzentration PCT [ng/mL]** | |
|---|---|---|
| | < 300 nm | > 350 nm |
| **Mittelwert [ng/mL] (Sollwert = 0,5 ng/mL)** | 1,01 | 0,60 |
| **St. Abweichung** | 0,0773 | 0,0294 |
| **VK %** | 7,65 | 4,86 |

### III. Ausführungsvarianten mit unterschiedlichen Zuckerkonzentrationen

Um den Einfluß unterschiedlicher Zuckerkonzentrationen zu untersuchen wurden Polymerpartikel mit Chlormethylgruppen, die eine durchschnittliche Partikelgröße von > 350 nm aufwiesen, in Lagerpuffern mit unterschiedlichen Zuckerkonzentrationen analysiert.

Figur 7 zeigt, dass ein Vergleich 50 g/L vs. 75 g/L vs. 125 g/L Saccharose im Lagerpuffer ähnliche Kalibrationskurven liefert. Allerdings wird die Präzision (gemessen als VK% von 20-fach Bestimmung einer Probe mit 0,5 ng/mL PCT) deutlich besser (VK% niedriger) je höher die Saccharose-Konzentration ist (vgl. Tabelle 3 unten).

**Tabelle 3**

| | **Saccharose 50 g/L** | **Saccharose 75 g/L** | **Saccharose 125 g/L** |
|---|---|---|---|
| **Präzision [VK %]** | 13,97 | 12,53 | 6,53 |

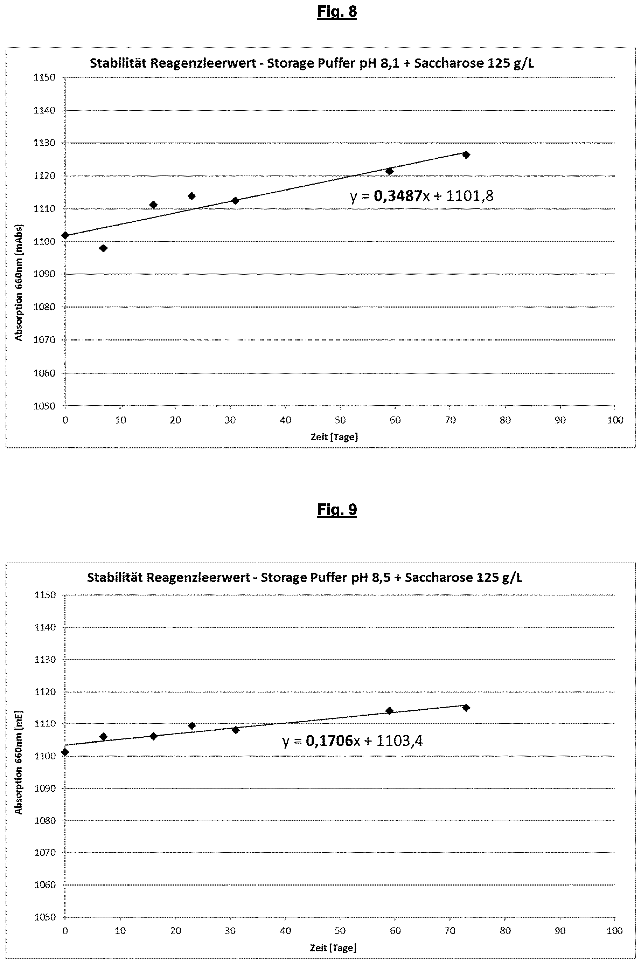

Die Figuren 8, 9 und 10 zeigen Daten des kombinierten Effektes Saccharose + pH-Wert. Die Chloromethylpartikel werden in drei Lagerpuffern mit Saccharose 125 g/L und pH 8,1 / 8,5 und 9,0 resuspendiert. Die Steigung der Gerade der linearen Regression der Reagenzleerwerte an den verschiedenen Messtagen entspricht der Aggregation der Partikel und soll so nah an 0 liegen wie möglich (Excel Folie "Kombinierter Effekt Sacc-pH").

### IV. Ausführungsvariante mit Carboxylgruppen

Die eingesetzten Polymerpartikel wiesen eine durchschnittliche Partikelgröße von < 310 nm auf. Die Partikel wurden über einen Zeitraum von 12 bis 24 Stunden mit den Antikörpern gekoppelt und der pH-Wert bei der Kopplungsreaktion betrug 4 bis 6.

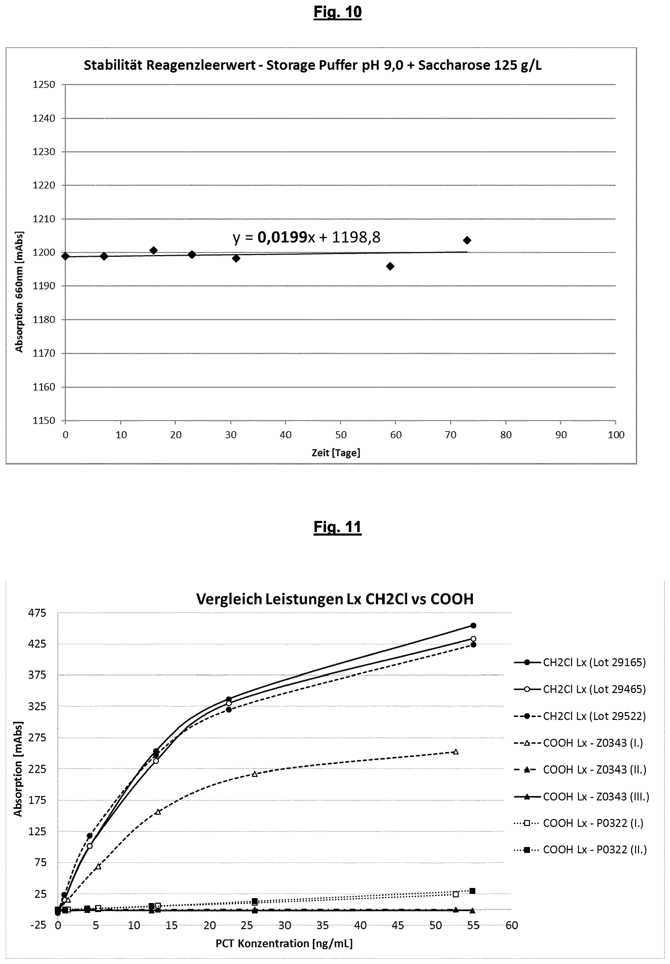

Wie die in Figur 11 dargestellten Ergebnisse zeigen, kann mit Polymerpartikeln mit Carboxylgruppe eine gute Reaktivität erreicht werden. Im Vergleich hierzu weisen die Polymerpartikel mit Chloromethylgruppen jedoch eine deutlich stärkere Reaktivität auf.

Polymerpartikel mit Carboxylgruppe müssen außerdem vor der Kopplungsreaktion aktiviert werden (EDC+NHS), um die COOH-Gruppen reaktiv zu machen. Diese Aktivierung ist komplex und funktioniert nicht immer reproduzierbar und Chargen-homogen. Im vorliegenden Fall war von fünf Versuchen nur eine Aktivierung tatsächlich erfolgreich. Im Vergleich hierzu koppeln Polymerpartikel mit Chloromethylgruppen sehr zuverlässig (hier die Daten von drei Ansätzen).

## Patentansprüche

1. Diagnostisches Reagenz zur quantitativen Bestimmung von Procalcitonin in einer Probe, wobei das Reagenz eine wässrige Suspension von Polymerpartikeln mit kovalent daran gebundenen Antikörpern gegen Procalcitonin ist, **dadurch gekennzeichnet, dass**
- die suspendierten Polymerpartikel eine durchschnittliche Partikelgröße im Bereich von >300 nm bis 450 nm aufweisen,
- die Suspension einen Anteil an darin gelöstem Zucker oder Zuckeralkohol im Bereich von 25 bis 250 g/l aufweist, und
- die Suspension einen pH-Wert im Bereich von 8,5 bis 10 aufweist.

2. Diagnostisches Reagenz nach Anspruch 1, **dadurch gekennzeichnet, dass** das Polymer der Polymerpartikel ausgewählt ist unter Acrylpolymer, Dextran-Epichlorhydrin-Copolymer, Polymethylmethacrylat, Polystyrol und Silica.

3. Diagnostisches Reagenz nach einem der Ansprüche 1 und 2, **dadurch gekennzeichnet, dass** die kovalente Bindung der Antikörper über an der Oberfläche der Polymerpartikel angeordnete funktionelle Gruppen ausgebildet ist, wobei die funktionellen Gruppen ausgewählt sind unter einer Carboxylgruppe (-COOH), primären Amingruppe (-RNH₂), aromatischen Amingruppe (-ArNH₂), Chlormethylgruppe (-CH₂Cl),einer aromatischen Chlormethylgruppe (-ArCH₂Cl), Amidgruppe (-CONH₂), Hydrazidgruppe (-CONHNH₂), Aldehydgruppe (-CHO), Hydroxylgruppe (-OH), Thiolgruppe (-SH), Epoxygruppe und Biotin-Avidin.

4. Diagnostisches Reagenz nach einem der Ansprüche 1 bis 3, **dadurch gekennzeichnet, dass** der Zucker oder Zuckeralkohol ausgewählt ist unter Saccharose, Mannitol, Sorbitol, Xylitol, Maltitol, Raffinose, Rhamnose und Kombinationen hiervon.

5. Diagnostisches Reagenz nach einem der Ansprüche 1 bis 4, **dadurch gekennzeichnet, dass** die Absorption der Suspension bei 660 nm innerhalb von 90, 120, 150 oder 180 Tagen oder gar innerhalb von 12 Monaten oder 24 Monaten ab dem Zeitpunkt der Herstellung der Suspension um weniger als 5%, weniger als 3% oder gar weniger als 2% vom Ausgangswert am Tag 0 abweicht.

6. Diagnostisches Reagenz nach einem der Ansprüche 1 bis 5, **dadurch gekennzeichnet, dass** die an den Polymerpartikeln kovalent gebundenen Antikörper gegen Procalcitonin monoklonale polyklonale oder rekombinante Antikörper oder Antikörper-Fragmente sind.

7. Diagnostisches Reagenz nach einem der Ansprüche 1 bis 6, **dadurch gekennzeichnet, dass** die Suspension einen pH-Wert im Bereich von 9,0 bis 10,0 aufweist.

8. Verfahren zur Herstellung eines diagnostischen Reagenz nach einem der Ansprüche 1 bis 7, **dadurch gekennzeichnet, dass** die kovalente Bindung der Antikörper über die funktionellen Gruppen an der Oberfläche der Polymerpartikel bei einem pH-Wert von 3 bis 6 erfolgt.

9. Verfahren nach Anspruch 8, **dadurch gekennzeichnet, dass** die kovalente Bindung der Antikörper über die funktionellen Gruppen an der Oberfläche der Polymerpartikel bei einer Temperatur im Bereich von 20 bis 29°C, im Bereich von 30 bis 34°C oder im Bereich von 35 bis 45°C erfolgt.

10. Verfahren nach einem der Ansprüche 8 und 9, **dadurch gekennzeichnet, dass** die kovalente Bindung der Antikörper in einem definierten pH-Bereich und bei einer definierten Temperatur über einen Zeitraum von 20 bis 80 Stunden erfolgt.

11. Diagnostisches Reagenz zur quantitativen Bestimmung von Procalcitonin in einer Probe, **dadurch gekennzeichnet, dass** es nach einem Verfahren nach einem der Ansprüche 8 bis 10 erhältlich ist.

## Claims

1. Diagnostic reagent for quantitative determination of procalcitonin in a sample, wherein the reagent is an aqueous suspension of polymer particles with covalently bound antibodies against procalcitonin, **characterised in that**
- the suspended polymer particles have an average particle size in the range from >300 nm to 450 nm,
- the suspension comprises a proportion of sugar or sugar alcohol dissolved therein in the range from 25 to 250 g/l, and
- the suspension has a pH in the range from 8.5 to 10.

2. Diagnostic reagent according to claim 1, **characterised in that** the polymer of the polymer particles is selected from acrylic polymer, dextran-epichlorohydrin copolymer, polymethyl methacrylate, polystyrene and silica.

3. Diagnostic reagent according to any one of claims 1 and 2, **characterised in that** the covalent bond of the antibodies is formed via functional groups disposed on the surface of the polymer particles, wherein the functional groups are selected from a carboxyl group (-COOH), primary amine group (-RNH₂), aromatic amine group (-ArNH₂), chloromethyl group (-CH₂Cl),an aromatic chloromethyl group (-ArCH₂Cl), amide group (-CONH₂), hydrazide group (-CONHNH₂), aldehyde group (-CHO), hydroxyl group (-OH), thiol group (-SH), epoxy group and biotin-avidin.

4. Diagnostic reagent according to any one of claims 1 to 3, **characterised in that** the sugar or sugar alcohol is selected from sucrose, mannitol, sorbitol, xylitol, maltitol, raffinose, rhamnose, and combinations thereof.

5. Diagnostic reagent according to any one of claims 1 to 4, **characterised in that** the absorbance of the suspension at 660 nm within 90, 120, 150 or 180 days or even within 12 months or 24 months from the time of preparation of the suspension deviates by less than 5%, less than 3% or even less than 2% from the initial value on day 0.

6. Diagnostic reagent according to any one of claims 1 to 5, **characterised in that** the antibodies against procalcitonin covalently bound to the polymer particles are monoclonal polyclonal or recombinant antibodies or antibody fragments.

7. Diagnostic reagent according to any one of claims 1 to 6, **characterised in that** the suspension has a pH in the range from 9.0 to 10.0.

8. Method for preparing a diagnostic reagent according to any one of claims 1 to 7, **characterised in that** the covalent binding of the antibodies via the functional groups on the surface of the polymer particles takes place at a pH of 3 to 6.

9. Method according to claim 8, **characterised in that** the covalent binding of the antibodies via the functional groups on the surface of the polymer particles takes place at a temperature in the range from 20 to 29°C, in the range from 30 to 34°C or in the range from 35 to 45°C.

10. Method according to any one of claims 8 and 9, **characterised in that** the covalent binding of the antibodies takes place in a defined pH range and at a defined temperature over a period of 20 to 80 hours.

11. Diagnostic reagent for quantitative determination of procalcitonin in a sample, **characterised in that** it is obtainable by a method according to any one of claims 8 to 10.

## Revendications

1. Réactif de diagnostic permettant de déterminer la quantité de procalcitonine dans un échantillon, dans lequel le réactif est une suspension aqueuse de particules de polymère auxquelles des anticorps anti-procalcitonine sont liés de manière covalente, **caractérisé en ce que**
- les particules de polymère en suspension présentent une taille moyenne de particules dans la plage comprise entre > 300 nm et 450 nm,
- la suspension contient une fraction de sucre ou de sucre-alcool dissous dans la plage comprise entre 25 et 250 g/l, et
- la suspension présente une valeur de pH dans la plage comprise entre 8,5 et 10.

2. Réactif de diagnostic selon la revendication 1, **caractérisé en ce que** le polymère des particules de polymère est choisi parmi un polymère d'acryle, un copolymère dextrane-épichlorhydrine, un polyméthacrylate de méthyle, un polystyrène et de la silice.

3. Réactif de diagnostic selon l'une des revendications 1 et 2, **caractérisé en ce que** la liaison covalente des anticorps est formée par l'intermédiaire de groupes fonctionnels agencés à la surface des particules de polymère, les groupes fonctionnels étant choisis parmi un groupe carboxyle (-COOH), un groupe amine primaire (-RNH₂), un groupe amine aromatique (-ArNH₂), un groupe chlorométhyle (-CH₂Cl),un groupe chlorométhyle aromatique (-ArCH₂Cl), un groupe amide (-CONH₂), un groupe hydrazide (-CONHNH₂), un groupe aldéhyde (-CHO), un groupe hydroxyle (-OH), un groupe thiol (-SH), un groupe époxy et un système biotine-avidine.

4. Réactif de diagnostic selon l'une des revendications 1 à 3, **caractérisé en ce que** le sucre ou le sucre-alcool est choisi parmi le saccharose, le mannitol, le sorbitol, le xylitol, le maltitol, le raffinose, le rhamnose et leurs combinaisons.

5. Réactif de diagnostic selon l'une des revendications 1 à 4, **caractérisé en ce que** l'absorption de la suspension à 660 nm diffère de la valeur initiale au jour 0 de moins de 5 %, de moins de 3 % ou de moins de 2 % dans les 90, 120, 150 ou 180 jours, voire dans les 12 mois ou 24 mois à compter de la date de préparation de la suspension.

6. Réactif de diagnostic selon l'une des revendications 1 à 5, **caractérisé en ce que** les anticorps anti-procalcitonine liés de manière covalente aux particules de polymère sont des anticorps ou des fragments d'anticorps monoclonaux, polyclonaux ou recombinants.

7. Réactif de diagnostic selon l'une des revendications 1 à 6, **caractérisé en ce que** la suspension présente une valeur de pH située dans la plage comprise entre 9,0 et 10,0.

8. Procédé de préparation d'un réactif de diagnostic selon l'une des revendications 1 à 7, **caractérisé en ce que** la liaison covalente des anticorps s'effectue par l'intermédiaire des groupes fonctionnels à la surface des particules de polymère à une valeur de pH comprise entre 3 et 6.

9. Procédé selon la revendication 8, **caractérisé en ce que** la liaison covalente des anticorps s'effectue par l'intermédiaire des groupes fonctionnels à la surface des particules de polymère à une température située dans la plage comprise entre 20 et 29°C, dans la plage comprise entre 30 et 34°C ou dans la plage comprise entre 35 et 45°C.

10. Procédé selon l'une des revendications 8 et 9, **caractérisé en ce que** la liaison covalente des anticorps s'effectue dans une plage de pH définie et à une température définie pendant une période comprise entre 20 et 80 heures.

11. Réactif de diagnostic permettant de déterminer la quantité de procalcitonine dans un échantillon, **caractérisé en ce qu'**il peut être obtenu grâce à un procédé selon l'une des revendications 8 à 10.
